# EUROPÄISCHE PATENTSCHRIFT

(11) **EP 0 484 333 B1**
(45) Veröffentlichungstag und Bekanntmachung des Hinweises auf die Patenterteilung: **15.11.1995**
(21) Anmeldenummer: 90906204.4
(22) Anmeldetag: 23.04.1990
(51) Int. Cl.: C07H 19/04, A61K 31/70, C12Q 1/68

(54) **NUCLEOSID-DERIVATE UND DEREN VERWENDUNG ALS ARZNEIMITTEL**
NUCLEOSIDE DERIVATIVES AND THEIR USE AS MEDICAMENTS
DERIVES DE NUCLEOSIDES ET LEUR UTILISATION COMME MEDICAMENTS

(30) Priorität: 24.07.1989 DE 3924424
(43) Veröffentlichungstag der Anmeldung: 13.05.1992
(60) Teilanmeldung: 95106043.3
(73) Patentinhaber: BOEHRINGER MANNHEIM GMBH, 68298 Mannheim (DE)
(72) Erfinder: SEELA, Frank, D-4500 Osnabrück (DE); BOURGEOIS, Werner, D-4500 Osnabrück (DE); GUMBIOWSKI, Rainer, D-4500 Osnabrück (DE); RÖLING, Angelika, D-4400 Münster (DE); ROSEMEYER, Helmut, D-4500 Osnabrück (DE); MERTENS, Alfred, D-6905 Schriesheim (DE); ZILCH, Harald, D-6800 Mannheim 31 (DE); KÖNIG, Bernhard, D-8137 Berg 3 (DE); KOCH, Edith, D-8122 Penzberg (DE)
(74) Vertreter: Weber, Manfred, Dr.
(86) Internationale Anmeldenummer: EP9000650
(87) Internationale Veröffentlichungsnummer: WO9101325

(56) Entgegenhaltungen:
- EP-A- 0 038 569
- EP-A- 0 043 722
- DE-A- 1 795 481
- GB-A- 696 952
- GB-A- 1 176 419
- GB-A- 1 474 299
- CHEMICAL ABSTRACTS, Band 105, Nr. 3, 21. Juli 1986, (Columbus, Ohio, US), N.B.DYATKINA et al.: "Nucleosides of Fluoro sugars. XV. Synthesis of 2',3'-Dedeoxy-3'-fluoro-D-Ribofuranosyl Benzimidazole - a new fluorosugar purine nucleoside analog", siehe seite 670* Zusammenfassung 24554w, & Z.Chem. 1985, 25(5), 180*
- Synthesis, Journal of Synthetic Organic Chemistry, Nr. 4, April 1985, Georg Thieme Verlag, (Stuttgart, DE), N.B. DYATKINA et al.: "Aminonucleotides and their derivatives: XIII. Synthesis of benzimidazole azidonucleotides", seiten 410-411
- CHEMICAL ABSTRACTS, Band 101, Nr. 25, 17. Dezember 1984, (Columbus, Ohio, US),J.S. SCHANCHE et al.: "Inactivation and reactivation of intracellular S-adenosyl-homocysteinase in the presence of nucleoside analogs in rat hepatocytes", siehe seite 27* Zusammenfassung 222195k, & Cancer Res. 1984,44(10), 4297-302*
- CHEMICAL ABSTRACTS, Band 102, Nr. 21, 27. Mai 1985, (Columbus, Ohio, US), T.ISHIKURA et al.: "Studies on the chemical synthesis of potential anti-metabolites. 37. Synthesis of 3-deazaadenine nucleosides modified at the sugar moiety", siehe seiten 613-614* Zusammenfassung 185416f, & Nucleosides Nucleotides 1984, 3(4), 413-22*
- Journal of Medicinal Chemistry, Band 25, Nr. 1, Januar 1982, American Chemical Society, J.A. MONTGOMERY et al.: "1-beta-D-Arabino-furanosyl-1H-imidazo (4,5-c)pyridine (ara-3-deazaadenine)", seiten 96-98
- CHEMICAL ABSTRACTS, Band 86, Nr. 11, 14. Marz 1977, (Columbus, Ohio, US),HUYNGH DINH TAM et al.: "Direct glycosylation of the indole nucleus with 2-deoxyribose", siehe seite 652* Zusammenfassung 73020s, & C.R. Hebd. SeancesAcad. Sci., Ser. C 1976, 283(5), 227-8*
- Journal Carbohydrates, Nucleosides, Nucleotides, Band 6, Nr. 5, 1979, Marcel Dekker, Inc., D.L. SWEENEY et al.: "Synthesis of a benzimidazole deoxyribodinucleotide", seiten 387-409
- CHEMICAL ABSTRACTS, Band 81, Nr. 5, 5. August 1974, (Columbus, Ohio, US), M.N.PREOBRAZHENSKAYA et al.: "1-beta-D-glucopyranosides of pyrrolo (2,3-b)pyridines (7-azaindoles)", siehe seite 454* Zusammenfassung 25898j, & Zh. Org. Khim. 1974, 10(4), 745-50*
- CHEMICAL ABSTRACTS, Band 90, Nr. 5, 29. Januar 1979, (Columbus, Ohio, US), J.A.MONTGOMERY et al.: "7-Amino-3-beta-D-ribofuranosyl-3H-1,2,3-triazolo (4,5-d)pyrimidine (8-azaadenosine). Nucleoside formation catalyzed by molecular sieve", siehe seite 515* Zusammenfassung 39168u, & Nucleic Acid Chem. 1978, 2,687-91*
- CHEMICAL ABSTRACTS, Band 84, Nr. 17, 26. April 1976, (Columbus, Ohio, US), G.R.REVANKAR et al.: "Synthesis and biological activity of some nucleosides resembling guanosine: imidazo (1,2-a) pyrimidine nucleosides", siehe seite 579*Zusammenfassung 122194w, & Ann. N.Y. Acad. Sci. 1975, 255 (Chem., Biol., Clin.Uses Nucleoside Analogs), 166-76*

## Beschreibung

Die Erfindung betrifft Nucleosid- und Nucleotid-Derivate, Verfahren zur Herstellung dieser Verbindungen, die Verwendung dieser Nucleosid-Derivate als Arzneimittel sowie deren Verwendung bei der Sequenzierung von Nucleinsäuren.

Gegenstand der vorliegenden Erfindung sind neue Nucleosid- und Nucleotid-Derivate der allgemeinen Formel I 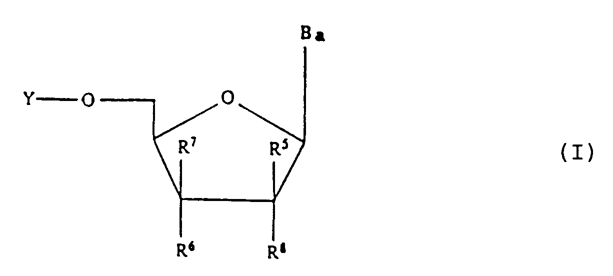
in der
- Ba: eine Indolylgruppe 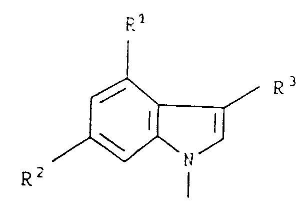 bedeutet,
wobei
- R¹: Wasserstoff, eine Amino- C₁-C₆-Alkoxy-, Halogen- oder Nitrogruppe,
- R²: Wasserstoff oder eine Halogen- oder Aminogruppe,
- R3: Wasserstoff und
- R⁵, R⁶, R⁷ und R⁸: jeweils Wasserstoff oder R⁵ und R⁷ zusammen eine weitere Bindung zwischen C-2' und C-3' darstellen können, und
- Y: Wasserstoff oder eine C₁-C₇-Alkylcarbonyl-, Monophoshat-, Diphosphat- oder Triphosphatgruppe darstellt
sowie deren mögliche α- oder β-Anomere, Tautomere und Salze.

Die Verbindungen der allgemeinen Formel I sind neue Verbindungen.

Ähnliche Verbindungen der Formel I sind aus EP-A-286,028 bekannt. Die vorliegenden erfindungsgemäßen Verbindungen unterscheiden sich jedoch strukturell von den bekannten Verbindungen durch den Indolylrest. Weiterhin weisen sie überraschende und überlegene Eigenschaften bzgl. der Verwendung als Arzneimittel auf.

Nucleosid-Derivate mit einem Indolylrest als Base sind aus GB-A-1176419 und T. Huynh et al., C. R. Acad. Sc. Paris t. 283 Serie C, 227 (1976) bekannt, jedoch besitzen die dort genannten Verbindungen im Gegensatz zu den erfindungsgemäßen einen Ribose- bzw. 2'-Desoxyribose-Zuckerrest.

Die "Alkyl-"teile in der Definition des Substituenten R¹ können geradkettig oder verzweigt sein und 1 - 6, vorzugsweise 1 - 4 Kohlenstoffatome enthalten. Ganz besonders bevorzugt sind die Methyl- und die Ethylgruppe.

Unter Halogen in der Definition der Substituenten R¹ und R² werden Fluor, Chlor, Brom und Jod verstanden, besonders bevorzugt sind das Fluor- und Chloratom.

Die Monophosphatgruppe ist die Gruppe -PO(OH)₂, die Diphosphatgruppe die Gruppe -P₂O₃(OH)₃ und die Triphosphatgruppe bedeutet die Gruppe -P₃O₅(OH)₄.

Als mögliche Salze kommen vor allem Alkali-, Erdalkali- und Ammoniumsalze der Phosphatgruppen in Frage. Als Alkalisalze sind Lithium-, Natrium- und Kaliumsalze bevorzugt. Als Erdalkalisalze kommen insbesondere Magnesium- und Calciumsalze in Frage. Unter Ammoniumsalzen werden erfindungsgemäß Salze verstanden, die das Ammoniumion enthalten, das bis zu vierfach durch Alkylreste mit 1 - 4 Kohlenstoffatomen oder/und Aralkylreste, bevorzugt Benzylreste, substituiert sein kann. Die Substituenten können hierbei gleich oder verschieden sein. Die Salze der Phosphate können auf bekannte Weise in die freien Säuren überführt werden.

Die Verbindungen der Formel I können basische Gruppen, insbesondere Amino-Gruppen enthalten, die mit geeigneten Säuren in Säureadditionssalze überführt werden können. Als Säuren kommen hierfür beispielsweise in Betracht: Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure.

Besonders bevorzugte Verbindungen der allgemeienen Formel I sind solche, die als Glyceropentofuranoside bezeichnet werden, insbesondere deren Didesoxy- und Didesoxy-didehydro-Derivate.

Für R¹-R³ und R⁵-R⁸ kommen insbesondere die folgenden Gruppen in Frage: für R¹ Wasserstoff, Amino, Chlor, C₁-C₆-Alkoxy, insbesondere Methoxy oder Nitro; für R² Wasserstoff oder Amino; für R³ Wasserstoff; für R⁵-R⁸ Wasserstoff bzw. R⁵ und R⁷ bilden gemeinsam eine Bindung (2',3'-Didesoxy-2',3'-didehydroribofuranosyl-Derivate).

R¹ bedeutet vorzugsweise eine Amino- oder Nitrogruppe und R², R³, R⁵-R⁸ jeweils ein Wasserstoffatom, bzw. R5 und R7 zusammen auch eine Bindung.

Die erfindungsgemäßen Verbindungen können in Analogie zu bekannten, verwandten Verbindungen hergestellt werden. Als besonders zweckmäßig hat sich zur Herstellung der Verbindungen der Formel I ein Verfahren erwiesen, bei dem man die Verbindung der Formel II

Ba-X (II)

in der
Ba die oben angegebene Bedeutung hat, und X Wasserstoff oder eine Alkalimetallgruppe, wie z.B. Lithium oder Natrium bedeutet,
mit einer Verbindung der Formel III 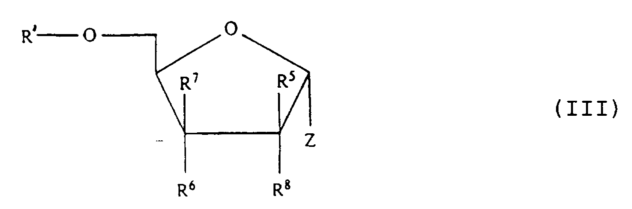
in der
- R⁵, R⁶, R⁷ und R⁸: die oben angegebene Bedeutung haben,
- R': eine Sauerstoffschutzgruppe und
- Z: eine reaktive Gruppe bedeutet,
zu einer Verbindung der Formel IV 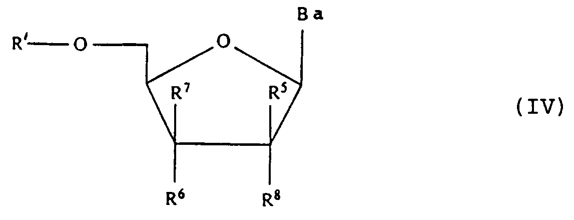
in der
Ba, R¹, R², R³, R⁵, R⁶, R⁷, R⁸ und R' die oben angegebene Bedeutung haben,
umsetzt und gegebenenfalls vorhandenen Schutzgruppen abspaltet
und gewünschtenfalls anschließend Verbindungen der Formel I, in denen Y Wasserstoff bedeutet, in bekannter Weise in die Mono-, Di- oder Triphosphate überführt
und gewünschtenfalls erhaltene freie Basen bzw. Säuren in die entsprechenden Salze oder erhaltene Salze in die entsprechenden freien Basen bzw. Säuren umwandelt.

Die Verbindungen der Formel II werden mit den Verbindungen der Formel III umgesetzt, besonders vorteilhaft unter Phasentransferbedingungen. Unter den Bedingungen der Phasentransferkatalyse werden die Basen der Formel II in ein entsprechendes Anion überführt, beispielsweise durch 50 %ige wässrige Natronlauge. Das so entstandene Anion wird durch einen Phasentransferkatalysator, beispielsweise Tris[2-(2-methoxyethoxy)ethyl]amin, hydrophobiert und in die organische Phase transportiert, in der es mit der reaktiven Verbindung der Formel III abreagiert.

Als reaktive Gruppen Z in den Verbindungen der allgemeinen Formel III kommen vorzugsweise Halogen, Acetoxy und Alkoxygruppen in Frage. Die Hydroxygruppen des Zuckerrestes werden bei dieser Umsetzung in üblicher Weise durch dem Fachmann geläufige Sauerstoffschutzgruppen, beispielsweise Toluoyl-, Benzoyl-, Tetrahydropyranyl- oder Acetylgruppen, geschützt. Die Sauerstoffschutzgruppen können nach beendeter Umsetzung in bekannter Weise unter alkalischen Bedingungen wieder abgespalten werden, zweckmäßigerweise verwendet man eine 1M methanolische Methanolatlösung.

Es kann zweckmäßig sein, auch die Reste R¹, R² und R³ während der Reaktion durch geeignete Schutzgruppen geschützt zu halten.

Eine weitere vorteilhafte Methode zur Herstellung der Verbindungen der Formel IV stellt das FestXFlüssig-PhasentransferVerfahren unter Verwendung von festem, pulverförmigem Kaliumhydroxid, dem oben genannten Kryptanden, sowie den Verbindungen der Formel II und III in einem aprotischen Lösungsmittel dar.

Verbindungen der Formel I, in denen R⁵, R⁶, R⁷ oder R⁸ eine Hydroxygruppe bedeuten, können nach vorherigem Schutz der 5'-Hydroxygruppe nach bekannten Methoden desoxygeniert werden, wobei Verbindungen der Formel I entstehen, in denen R⁵, R⁶, R⁷ oder R⁸ Wasserstoff bedeuten. Zum selektiven Schutz der Hydroxygruppe in 5'-Stellung stehen dem Fachmann bekannte Verfahren zur Verfügung. Beispielsweise hat sich in der Nucleotidchemie die 4,4'-Dimethoxytriphenylmethylgruppe bewährt. Diese kann nach erfolgter Umsetzung wieder leicht durch milde Säurehydrolyse abgespalten werden, während die ebenfalls säurelabile glycosidische Bindung unter diesen Bedingungen nicht hydrolisiert wird. Die Umsetzung des zu schützenden Nucleosids mit dem Sauerstoffschutzgruppenreagenz für die 5'-Hydroxygruppe wird in einem geeigneten organischen Lösungsmittel, zweckmäßigerweise getrocknetem Pyridin, mit einem leichten Überschuß des Sauerstoffschutzgruppenreagenzes sowie gegebenenfalls einer geeigneten Hilfsbase, beispielsweise N-Ethyldiisopropylamin, durchgeführt.

Zur Desoxygenierung wird die Verbindung der allgemeinen Formel I, in der R⁵, R⁶, R⁷ oder R⁸ eine Hydroxygruppe vorstellt und bei der die 5'-Hydroxygruppe in vorstehender Weise geschützt worden ist und auch sonstige funktionelle Reste Schutzgruppen tragen, zunächst in ein 2'- oder 3'-O-Thiocarbonylderivat überführt, welches anschließend mit Tributylzinnhydrid radikalisch reduzirt wird. Solche Methoden zur Desoxygenierung von 2'-Desoxynucleosiden zu 2'- und 3'-Didesoxynucleosiden sind dem Fachmann bekannt. Als besonders günstig hat sich die Methode der Barton-Desoxygenierung erwiesen (J. Chem. Soc., Perkin Trans. I (1975) 1574).

Verbindungen der Formel I, in denen R⁵ und R⁷ eine weitere Bindung Zwischen C-2' und C-3' darstellen, können in Analogie zu bekannten verwandten Verbindungen (Robins, Hansske Tetrahedron Letters, 25, 367, 1984 und hierin zitierte Literatur) hergestellt werden. Als besonders zweckmäßig hat sich zur Herstellung dieser Verbindungen ein Verfahren erwiesen, bei dem man die entsprechenden Ribosen mit Acetoxyisobutyrylbromid umsetzt und die entstandenen Isomeren anschließend mit einem Reduktionsmittel wie z.B. dem Zink/Kupfer Paar oder ähnlichen Reduktionsmitteln reduziert und aus dem erhaltenen Rohprodukt nach Abspaltung der Schutzgruppe unter alkalischen Bedingungen die 2',3'-Didesoxy-2',3'-didehydro-Derivate erhält.

Neben diesem Verfahren sind in der Literatur weitere Verfahren zur Didesoxygenierung und gleichzeitigen Einführung der Doppelbindung beschrieben (vgl. Jain et al. J.Org.Chem. 39, 30, 1974; Robins et al. JACS 98, 8204 und 8213, 1976; Adachi et al. J.Org.Chem. 44, 1404, 1979; Mengel et al. Tetrah. Lett. 4203, 1977; Classon et al. Acta Chem. Scand. B 36, 251, 1982; Chu et al. J.Org.Chem. 54, 2217, 1989). Weiterhin können diese Verbinungen aus entsprechenden 2'-Desoxyribosen nach bekannten Verfahren (vgl. Horwitz et al. JACS 86, 1896, 1964; McCarthy et al. JACS 88, 1549, 1966; Samukov et al. Biorg. Khim. 9, 52, 1982) der Monodesoxygenierung unter gleichzeitiger Einführung der Doppelbindung hergestellt werden. Ein weiterer Weg zur Herstellung dieser Verbindungen ist die Umsetzung einer 2',3'-Didesoxy-2',3'-didehydroribose mit einem entsprechend substituerten Basenderivat Ba wie sie einem Fachmann aus der Literatur (vgl. z.B. EP-A-0,286,028 ) bekannt ist.

Die Phosphatgruppen werden in Verbindungen der allgemeinen Formel I, in denen Y Wasserstoff bedeutet, in bekannter Weise eingeführt. Die Monophosphate erhält man beispielsweise, indem man Verbindungen der Formel I, in denen Y Wasserstoff bedeutet, mit Phosphoroxychlorid in Trimethylphosphat phosphoryliert. Die auf diesem Wege erhaltenen Triethylammoniumsalze können in bekannter Weise in andere Salze durch Umsalzen überführt werden. Die Di- bzw. Triphosphate werden nach bekannten Methoden, vorzugsweise aus den Monophosphaten durch Umsetzung mit o-Phosphaten bzw. Pyrophosphaten erhalten. Ihre verschiedenen Salze können ebenfalls nach bekannten Methoden hergestellt werden.

Die Verbindungen der Formel II sind bekannte Verbindungen oder können in Analogie zu bekannten Verbindungen hergestellt werden. Derartige Herstellungsverfahren sind beispielsweise beschrieben in Chem. Ber. 110 (1977) 1462, J. Chem. Soc. 1960, 131 bzw. Tetrahedron Lett. 21 (1980) 3135.

Auch die Verbindungen der Formel III sind zum Teil bekannte Verbindungen. Bisher nicht beschriebene Verbindungen können in völliger Analogie zu den bekannten Verbindungen hergestellt werden. Die Herstellung einer solchen Verbindung ist beispielsweise beschrieben in Chem. Ber. 93 (1960) 2777 bzw. Synthesis 1984, 961.

Die Verbindungen der vorliegenden Erfindung weisen wertvolle pharmakologische Eigenschaften auf. Insbesondere eignen sie sich zur Therapie und Prophylaxe von Infektionen, die durch DNA-Viren wie z.B. das Herpes-Simplex-Virus, das Zytomegalie-Virus, Papova-Viren, das Varicella-Zoster-Virus oder Epstein-Barr-Virus oder RNA-Viren wie Toga-Viren oder insbesondere Retroviren wie die Onko-Viren HTLV-I und II, sowie die Lentiviren Visna und Humanes-Immunschwäche-Virus HIV-1 oder -2, verursacht werden.

Besonders geeignet erscheinen die Verbindungen der Formel I zur Behandlung der klinischen Manifestationen der retro-viralen HIV-Infektion beim Menschen, wie der anhaltenden, generalisierten Lymphadenopathie (PGL), dem fortgeschrittenen Stadium des AIDS-verwandten Komplex (ARC) und dem klinischen Vollbild von AIDS.

Überraschenderweise wurde nun gefunden, daß Verbindungen der allgemeinen Formel I die Vermehrung von DNA- bzw. RNA-Viren auf der Stufe der virusspezifischen DNA- bzw. RNA-Transkription hemmen. Die Substanzen können speziell über die Inhibierung des Enzyms Reverse Transkriptase oder über einen Kettenabbruch der wachsenden DNA-Kette die Vermehrung von Retroviren beeinflussen (vgl. Proc. Natl. Acad. Sci. USA 83, 1911, 1986 bzw. Nature 325, 773 1987). Von besonderem therapeutischem Interesse ist die Hemmwirkung auf das HIV-Virus, dem Verursacher der Immunschwäche-Erkrankung AIDS. Zur Behandlung von AIDS ist heute nur 3'-Azido-3'-desoxythymidin ( DE-A-3608606) bei AIDS Patienten zugelassen. Jedoch machen toxische Nebenwirkungen des 3'-Azido-3'-desoxythymidins auf das Knochenmark bei etwa 50 % der behandelten Patienten Bluttransfusionen erforderlich. Die Verbindungen der allgemeinen Formel I besitzen diese Nachteile nicht. Sie wirken antiviral, ohne in pharmakologisch relevanten Dosen cytotoxisch zu sein.

Die erfindungsgemäßen Substanzen der Formel I lassen sich auch vorteilhaft bei der DNA-Sequenzierung nach Sanger einsetzen. Besonders die Sequenzierung d(G-C)-reicher DNA-Fragmente wird durch die Bildung von Sekundärstrukturen, die zu einer Bandenkompression im Bereich von d(G-C)-Clustern führen, erschwert.

Die erfindungsgemäßen Verbindungen der Formel I, in denen R⁶ und R⁷ Wasserstoff bedeuten, werden bei der DNA-Sequenzierung nach Sanger als Kettenterminatoren anstelle der bekannten 2',3'-Didesoxyverbindungen verwendet.

Nucleinsäuren, die als Baustein eine oder mehrere Verbindungen der Formel I enthalten, können nach bekannten Verfahren hergestellt werden (beispielsweise Nucleic Acids Research Vol. 14, Nr. 5, 1986, S. 2319 ff). Sie entstehen aber auch beispielsweise bei der DNA-Sequenzierung. Wird als Baustein eine Verbindung der Formel I verwendet, in der R⁶ Wasserstoff bedeutet, so kann ein solcher Baustein nur einmal, nämlich am Kettenende der Nucleinsäure eingebaut sein. Die erfindungsgemäßen Nucleinsäuren sind aus 2 bis 1000, vorzugsweise 8 bis 50 Nucleotidbausteinen aufgebaut. Besonders bevorzugt sind Nucleinsäuren mit 15 - 30 Nucleotidbausteinen.

Diese Nucleinsäuren können ebenfalls als antivirale Mittel eingesetzt werden. Als sogenannte anti-sense-Nucleinsäure hybridisiert diese Nucleinsäure mit der ssDNA/RNA des Virus und erschwert die Transkription zur Virus-DNA. Solche Nucleinsäuren können insbesondere als Mittel gegen AIDS verwendet werden, da sie nicht oder nur schwer durch zelleigene Restriktionsenzyme abgebaut werden.

Zur Herstellung von Arzneimitteln werden die Substanzen der allgemeinen Formel I, ihre pharmakologisch verträglichen Salze oder sie enthaltende Nucleinsäuren in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die erfindungsgemäßen Substanzen können in flüssiger oder fester Form enteral oder parenteral appliziert werden. Als Injektionsmedium kommt vorzugsweise Wasser zur Anwendung, welches die bei Injektionslösungen üblichen Zusätze, wie Stabilisierungsmittel, Lösungsvermittler oder Puffer enthält.

Derartige Zusätze sind z.B. Tartrat- und Citratpuffer, Ethanol, Komplexbildner (wie Ethylendiamintetraessigsäure und deren nichttoxischen Salze) und hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselsäuren, hochmolekulare Fettsäuren (wie Stearinsaure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die erfindungsgemäßen Verbindungen werden üblicherweise in Mengen von 0.1 - 100 mg, vorzugsweise 0.2 - 80 mg pro Tag und pro kg Körpergewicht appliziert. Bevorzugt ist es, die Tagesdosis auf 2 - 5 Applikationen zu verteilen, wobei bei jeder Applikation 1 - 2 Tabletten mit einem Wirkstoffgehalt von 0.5 - 1000 mg verabreicht werden. Die Tabletten können auch retardiert sein, wodurch sich die Anzahl der Applikationen pro Tag auf 1 - 3 vermindert. Der Wirkstoffgehalt der retardierten Tabletten kann 2 - 2000 mg betragen. Der Wirkstoff kann auch durch Injektion ein- bis achtmal pro Tag bzw. durch Dauerinfusion gegeben werden, wobei Mengen von 5 - 4000 mg/Tag normalerweise ausreichen.

Neben den in den Beispielen genannten Verbindungen kommen im Sinne der vorliegenden Erfindung beispielsweise die folgenden Verbindungen in Frage:
1-(2,3-Didesoxy-3-fluor-β-D-glyceropentofuranosyl)-4-amino-1H-indol
1-(2,3-Didesoxy-3-azido-β-D-glyceropentofuranosyl)-4-amino-1H-indol
1-(2,3-Didesoxy-β-D-glyceropentofuranosyl)-4,6-diamino-1H-indol
1-(2,3-Didesoxy-β-D-glyceropentofuranosyl)-6-hydroxy-1H-indol
1-(2,3-Didesoxy-β-D-glyceropentofuranosyl)-4-amino-6-hydroxy-1H-indol
1-(2,3-Didesoxy-β-D-glyceropentofuranosyl)-4-methylamino-1H-indol
1-(2,3-Didesoxy-β-D-glyceropentofuranosyl)-4,6-dihydroxy-1H-indol
1-(2,3-Didesoxy-β-D-glyceropentofuranosyl)-4-hydroxy-6-amino-1H-indol
1-(2,3-Didesoxy-β-D-glyceropentofuranosyl)-4-methyl-1H-indol
1-(2,3-Didesoxy-2,3-didehydro-β-D-glyceropentofuranosyl)-4-amino-6-chlor-1H-indol
1-(2,3-Didesoxy-2,3-didehydro-β-D-glyceropentofuranosyl)-4-mercapto-1H-indol
1-(2,3-Didesoxy-2,3-didehydro-β-D-glyceropentofuranosyl)-4-methylmercapto-1H-indol
1-(2,3-Didesoxy-2,3-didehydro-β-D-glyceropentofuranosyl)-4-methoxy-1H-indol
1-(2,3-Didesoxy-2,3-didehydro-β-D-glyceropentofuranosyl)-4-dimethylamino-1H-indol
1-(2,3-Didesoxy-2,2-difluoro-β-D-glyceropentofuranosyl)-4-amino-1H-indol
1-(2,3-Didesoxy-2-fluoro-β-D-arabinofuranosyl)-4-amino-1H-indol
1-(2,3-Didesoxy-2-azido-β-D-arabinofuranosyl)-4-amino-1H-indol
Die Erfindung wird durch die folgenden Beispiele näher erläutert:

### Beispiel 1

### 1-(2,3-Didehydro-2,3-didesoxy-β-D-glyceropentofuranosyl)-4-amino-1H-indol

a) 1-[2-Desoxy-3,5-di-O-(p-toluoyl)-β-D-erythro-pentofuranosyl]-4-nitro-1H-indol
Zu einer Lösung von 4-Nitroindol (972 mg, 6.0 mmol) in CH₃CN (50 ml) fügt man KOH (838 mg, 15.0 mmol) und TDA-1 (100 mg, 0.31 mmol) hinzu und rührt 15 Min. bei RT (N₂-Atmosphäre). Nach Zugabe der Halogenose (2.45 g, 6.3 mmol) rührt man weitere 15 Min. bei RT, filtriert, dampft bis zur Trockene ein und chromatographiert den Rückstand über eine kurze Kieselgel-60H-Säule. Eindampfen der Hauptzone und Kristallisation aus Benzol/Cyclohexan (1:2) liefert gelbe Nadeln (2.70 g, 85 %) (82%; N. S. Girgis, H. B. Cottam,and R. K. Robins, J. Heterocycl. Chem. 25, 361 (1988); Rf(CH₂Cl₂/ EtOAc 99:1)= 0.6; Rf(CH₂Cl₂)= 0.4.
¹H NMR ([D₆)DMSO): δ= 2.37, 2.41 (2s, 6H, 2 CH₃), 2.78 (m, 1H, H-2'b), 3.03 (m, 1H, H-2'a), 4.56 (m, 3H, H-4' und H-5'), 5.74 (m, 1H, H-3'), 6.75 (dd, J= 5.9 Hz u. 7.8 Hz, 1H, H-1'), 7.13 (d, J= 3.1 Hz, H-3), 7.29-7.40 (m, 5H, aromat. H), 7.83-8.02 (m, 5H, aromat. H und H-6), 8.11 (d, J= 7.9 Hz, 1H, H-5), 8.25 (d, J= 8.2 Hz, 1H, H-7).
b) 1-(2-Desoxy-β-D-erythro-pentofuranosyl)-4-nitro-1H-indol
Die unter a) erhaltene Verbindung (3.86 g, 7.5 mmol) wird mit MeOH (200 ml) versetzt und zusammen mit NaOMe/MeOH-Lösung (1M, 15.5 ml) 48 h bei Raumtemperatur gerührt. Nach Zugabe von Kieselgel 60 (5 g) dampft man bis zur Trockene ein und chromatographiert an Kieselgel 60H (Säule 3 x 30 cm, Laufmittel CHCl₃/MeOH 8:1, Rf= 0.3). Elution der Hauptzone ergibt nach Abdampfen des Lösungsmittels einen gelben Feststoff in 80% Ausbeute. (96%; N. S. Girgis, H. B. Cottam,and R. K. Robins, J. Heterocycl. Chem. 25, 361 (1988));
¹H NMR ([D₆]DMSO): δ= 2.31 (m, 1H, H-2'b), 2.51 (m,, 1H, H-2'a), 3.55 (m, 2H, H-5'), 3.87 (m, 1H, H-4'), 4.40 (m, 1H, H-3'), 4.97 (m, 1H, 5'-OH), 5.36 (m, 1H, 3'-OH), 6.51 (pt, J= 6.5 Hz, 1H, H-1'), 7.11 (d, J= 3.2 Hz, 1H, H-3), 7.36 (t, J= 8.1 Hz, 1H, H-6), 8.03 (d, J= 3.2 Hz, 1H, H-2), 8.10 (d, J= 8.0 Hz, 1H, H-5), 8.17 (d, J= 8.2 Hz, 1H, H-7).
c) 4-Amino-1-(2-desoxy-β-D-erythro-pentofuranosyl)-1H-indol (1,3,7-tridesaza-2'-desoxyadenosin)
1.O g der unter b) erhaltenen Verbindung wird in 50 ml Ethanol gelöst und in Gegenwart von 100 mg Pd/Kohle (10% Pd) 6 h bei RT und Normaldruck hydriert. Man filtriert, adsorbiert an Kieselgel und chromatographiert an Kieselgel 60 (Säule 15 x 3.5 cm). Man erhält einen farblosen Schaum (Ausbeute 70 %); (74%, N. S. Girgis, H. B. Cottam,and R. K. Robins, J. Heterocycl. Chem. 25, 361 (1988).
¹H NMR ([D₆]DMSO): δ= 2.17 (m, 1H, H-2'b), 2.42 (m, 1H, H-2'a), 3.51 (m, 2H, H-5'), 3.79 (m, 1H, H-4'), 4.32 (m, 1H, H-3'), 4.89 (m, 1H, 5'-OH), 5.26 (m, NH2 und 3'-OH), 6.23 (m, 2H, H-5 und H-1'), 6.59 (d, J= 3.3 Hz, 1H, H-3), 6.71 (d, J= 8.2 Hz, 1H, H-7), 6.84 (pt, J= 8.2 Hz, 1H, H-6), 7.31 (d, J= 3.3 Hz, 1H, H-2).
d) 4-{[(Dimethylamino)methylen]amino}-1-(2-desoxy-β-D-erythropentofuranosyl)-1H-indol
1.54 g (6.21 mmol) der unter c) erhaltenen Verbindung werden in 30 ml trockenem, aminfreiem Dimethylformamid gelöst und mit 12 ml (68.3 mmol) N,N-Dimethylformamiddiethylacetal versetzt. Die Reaktionsmischung wird 8 h bei 50 C unter Argon gerührt. Das Lösungsmittel wird im Vakuum abgedampft und der Rückstand mehrmals mit Toluol nachgedampft. Chromatographie an Kieselgel 60 H (Säule 13 x 5 cm, Laufmittel CHCl₃/MeOH 5:1) liefert 1.28 g (68 %) farbloses, schaumiges Produkt; DC (CHCl₃/MeOH 5:1): Rf= 0.4.
¹H NMR ([D₆)DMSO): δ= 2.19 (m, 1H, H-2'b), 2.46 (m, 1H, H-2'a), 3.01 (s, 6H, 2 CH₃, 3.51 (m, 2H, H-5'2), 3.81 (m, 1H, H-4'), 4.34 (m, 1H, H-3'), 4.89 (m, 1H, 5'-OH), 5.29 (d, J= 4.2 Hz, 1H, 3'-OH), 6.31 (dd, J= 6.2 und 7.6 Hz, 1H, H-1'), 7.00 (t, J= 7.8 Hz, 1H, H-6), 7.13 (d, J= 7.8 Hz, 1H, H- ), 7.43 (d, J= 3.4 Hz, 1H, H-2), 7.80 (s, 1H, CH).
e) 4-{[(Dimethylamino)methylen]amino}-1-[2-desoxy-5-O-(tert-butyldiphenylsilyl)-β-D-erythro-pentofuranosyl]-1H-indol
1.6 g (5.27 mmol) der unter d) erhaltenen Verbindung werden 2x mit Pyridin abgedampft und anschließend in Pyridin (26 ml) gelöst. In der Kälte wird TBDPSiCl (1.63 ml, 6.36 mmol) zugetropft und die Lösung 30 min. bei 0 C gerührt. Man läßt auf RT erwärmen und rührt weitere 24 h. Das Lösungsmittel wird abgedampft und der Rüchstand mit Toluol nachgedampft.
UV (MeOH): lₘₐₓ (ε)= 220 (44200), 298 nm (13200). ¹H NMR ([D₆]DMSO): δ= 0.87 (s, 9H, tBu), 2.12 (m, 1H, H-2'b), 2.41 (m, 1H, H-2'a), 2.86 (s, 6H, N(CH₃)₂), 3.64 (m, 2H, H-5'a,b), 3.78 (m, 1H, H-4'), 4.33 (m, 1H, H-3'), 5.22 (d, J= 4.5 Hz, 1H, 3'-OH), 6.20 (pt, J= 6.6 Hz, 1H, H-1'), 6.30 (d, J= 3.3 Hz, 1H, H-3), 6.36 (d, J= 7.4 Hz, 1H, H- ), 6.82 (t, J= 7.4 Hz, 1H, H-6), 7.01 (d, J= 7.4 Hz, 1H, H- ), 7.18 (d, J= 3.3 Hz, 1H, H-2), 7.20-7.50 (m, 10 phenyl-H), 7.64 (s, 1H, N=CH).

| | | | | |
|---|---|---|---|---|
| C₃₃H₃₉N₃O₃Si (553.78) | Ber. | C 71.57 | H 7.10 | N 7.59 |
| | Gef. | 71.45 | 7.21 | 7.72 |

f) 4-{[(Dimethylamino)methylen]amino}-1-[2-desoxy-5-O-(tert-butyldiphenylsilyl)-3-O-methylsulfonyl-β-D-erythro-pentofuranosyl]-1H-indol
800 mg (1.44 mmol) der unter e) erhaltenen Verbindung werden in CH₂Cl₂ (24 ml) gelöst und die Lösung mit Pyridin (5.5 ml) versetzt. Unter Eiskühlung wird Methansulfonylchlorid (2.17 ml, 28.5 mmol) zugetropft und die Mischung bei RT gerührt (4 h).
Nach Zugabe von MeOH (6.5 ml) wird mit CHCl₃ (100 ml) verdünnt, und mit 0.1 N HCl und H₂O (je 100 ml) extrahiert. Die org. Phase wird über Na₂SO₄ getrocknet, filtriert und das Lösungsmittel im Vakuum verdampft. Nach chromatographischer Aufarbeitung (Kieselgel 60 H) erhält man 310 mg (34%) eines farblosen Schaums.
¹H-NMR ([D₆]DMSO): δ= 1.02 (s, 9H, t-Bu), 2.62 (m, 1H, H-2'b), 2.57 (m, 1H, H-2'a), 3.00 (s, 6H, 2 CH₃), 3.32 (s, 3H, SCH₃), 3.82 (m, 2H, H-5'), 4.26 (m, 1H, H-4'), 5.47 (m, 1H, H-3'), 6.40 (dd, J= 6.1 und 8.1 Hz, 1H, H- 1'), 6.47 (d, J= 3.3 Hz, 1H, H-3), 6.51 (d, J= 7.5 Hz, 1H, ), 6.95 (pt, J= 7.5 Hz, 1H, H-6), 7.20 (d, J= 7.5 Hz, 1H, H- ), 7.40 (m, aromat. H und H-2), 7.64 (m, aromat. H), 7.78 (s, N=CH).
g) 4-[(Dimethylamino)methylen]amino-1-(2,3-didesoxy-2,3-didehydro-β-D-glycero-pentofuranosyl)-1H-indol
Die unter f) erhaltene Verbindung (420 mg, 0.84 mmol) wird in 50 ml THF gelöst und die Lösung mit 10 ml Bu₄NF (1M Lösung in THF) versetzt. Man rührt die Lösung 4 h bei 50 °C, verdampft das Lösungsmittel im Vakuum und adsorbiert den Rückstand an Kieselgel 60.
Nach Säulenchromatographie (30 x 3 cm, CHCl₃ - MeOH, (8:2) R_{f}=0.5 erhält man ein farbloses Öl.
¹H-NMR ([D₆]DMSO): δ = 3.01 (s, 6H, 2CH₃), 3.46 (m, 2H, H2-5'), 4.77 (m, 1H, H-4'), 4.91 (t, J=5,5 Hz, 1H, 5'-OH), 6.12 (m, 1H, H-2'), 6.49 (m, 3H, H-3' und H-7/H-5 und H-3), 7.00 (m, 2H, H-6 und H-1'), 7.19 (d, J=3.3 Hz, 1H, H-2), 7.23 (d, J=8.4 Hz, 1H, H-5/H-7), 7.81 (s, 1H, N=CH).
h) 4-Amino-(2,3-didehydro-2,3-didesoxy-β-D-glycero-pentofuranosyl)-1H-indol
Die unter g) erhaltene Verbindung (200 mg) wird in MeOH (5 ml) gelöst. Nach Zugabe von 20 ml conc. NH₃ (25%) kocht man die Lösung unter Rückfluß. Das Lösungsmittel wird im Vakuum verdampft und der Rückstand an Kieselgel chromatographiert (CHCl₃/MeOH, 8:2, R_{f}=0.84; CH₂Cl₂/MeOH, 95:5, R_{f}=0.40). ¹H-NMR ([D₆]DMSO): δ = 5.21 (s, NH₂), 6.91 (m, H-1'), 7.08 (d, J=3.4 Hz, H-2').

### Beispiel 2

### 1-(2,3-Didehydro-2,3-didesoxy-β-D-glyceropentofuranosyl)-4-nitro-1H-indol

a) 1-[2-Desoxy-5-O-(tert-butyldiphenylsilyl)-β-D-erythro-pentofuranosyl]-4-nitro-1H-indol
1.43 g (5.14 mmol) der unter Bsp. 1b erhaltenen Verbindung werden 2x mit Pyridin abgedampft, in 30 ml Pyridin gelöst, mit 1.57 ml (6.11 mmol) TBDPSiCl in der Kälte versetzt, 30 min. bei 0 C gerührt, 24 h bei RT gerührt, Py abgedampft, 2x mit Toluol nachgedampft, an Kieselgel 60 (15 g) adsorbiert. Chromatographie (Säule 20 x 5 cm), Rf (CH₂Cl₂)= 0.2, gelber Schaum, Ausbeute: 1.73 g (65 %).
UV (MeOH) : lₘₐₓ (ε)= 241 (Sch, 11900), 338 (4600), 372 (6100). ¹H NMR ([D₆]DMSO): δ= 0.96 (s, 9H, tBu), 2.30-2.64 (m, H-2'a,b), 3.79 (m, 2H, H-5'2), 3.97 (m, 1H, H- 4'), 4.53 (m, 1H, H-3'), 5.45 (d, J= 4.5 Hz, 1H, 3'-OH), 6.55 (pt, J= 6.4 Hz, 1H, H-1'), 7.03 (d, J= 3.1 Hz, 1H, H-3), 7.28-7.59 (m, 11 aromat. H), 7.90 (d, J= 3.1 Hz, 1H, H-2), 8.10 (d, J= 8.0 Hz, 1H, H-5), 8.15 (d, J= 8.2 Hz, 1H, H-7).

| | | | | |
|---|---|---|---|---|
| C₂₉H₃₂N₂O₅Si (516.67) | Ber. | C 67.42 | H 6.24 | N 5.42 |
| | Gef. | C 67.56 | H 6.23 | N 5.39 |

b) 1-[2-Desoxy-5-O-(tert-butyldiphenylsilyl)-3-O-methylsulfonyl-β-D-erythro-pentofuranosyl]-4-nitro-1H-indol
800 mg (1.55 mmol) der unter a) erhaltenen Verbindung werden in CH₂Cl₂ (26 ml)/Pyridin (6 ml) gelöst. Man versetzt unter Kühlung mit Methansulfonylchlorid (2.36 ml, 31 mmol), läßt die Mischung langsam auf RT erwärmen und rührt weitere 4 h. Man versetzt mit MeOH (1.7 ml), rührt weitere 15 Min., verdünnt mit CHCl₃ (100 ml) und extrahiert mit 0.1N HCl und H₂O (je 100 ml). Die organische Phase wird über Na₂SO₄ getrocknet. Das LM wird im Vakuum verdampft. Nach Chromatographie (CH₂Cl₂, Säule 30 x 4 cm, Rf 0.5) erhält man einen gelben Schaum (840 mg, 91%). UV (MeOH): lₘₐₓ (ε)= 235 (Sch, 13300), 270 (Sch, 1800), 338 (5300), 365 (6300), 388 (Sch, 5100).
¹H NMR ([D₆]DMSO): δ= 1.01 (s, 9H, t-Bu), 2.83 (m, 1H, H-2'b), 2.98 (m, 1H, H-2'a), 3.36 (s, 3H, S-CH₃), 3.86 (m, 2H, H-5'), 4.34 (m, 1H, H-4'), 5.53 (m, 1H, H-3'), 6.66 (pt, J= 6.2 Hz, 1H, H-1'), 7.08 (d, J= 3.3 Hz, 1H, H-3), 7.30-7.62 ( aromat. H sowie H-6), 7.94 (d, J= 3.3 Hz, 1H, H-2), 8.14 (d, J= 8 Hz, 1H, H-5), 8.23 (d, J= 8 Hz, 1H, H-7).

| | | | | | |
|---|---|---|---|---|---|
| C₃₀H₃₄N₂O₇SiS (594.76) | Ber. | C 60.58 | H 5.76 | N 4.71 | S 5.39 |
| | Gef. | 60.45 | 5.76 | 4.74 | 5.54 |

c) 1-(2,3-Didehydro-2,3-didesoxy-β-D-glycero-pentofuranosyl)-4-nitro-1H-indol
800 mg (1.35 mmol) der unter b) erhaltenen Verbindung werden in 25 ml THF gelöst, mit 5 ml Bu₄NF (1M Lsg.in THF) versetzt und die Lösung 2 h bei 50 °C unter N₂-Atmosphäre gerührt. Das LM wird im Vakuum abgedampft und der Rückstand an Kieselgel (Säule 30 x 3.5cm, Laufmittel CH₂Cl₂/MeOH 99:1, Rf 0.3) chromatographiert. Aus der Hauptzone erhält man nach Abdampfen des LM ein gelbes Öl, das beim Stehenlassen durchkristallisiert.
UV (MeOH): lₘₐₓ (ε)= 237 (11700), 339 (Sch, 4700), 370 (6100).
¹H NMR ([D₆]DMSO): δ= 3.51 (m, 2H, H-5'), 4.86 (m, 2H, H-4' und 5'-OH), 6.20 (pq, J= 6.0 Hz und 1.7 Hz, 1H, H-2'), 6.54 (pq, J= 6.0 Hz und J= 1.6 Hz, 1H, H-3'), 7.11 (d, J= 3.3 Hz, 1H, H-3), 7.21 (m, 1H, H-1'), 7.40 (t, J= 8.1 Hz, 1H, H-6), 7.81 (d, J= 3.3 Hz, 1H, H-2), 8.12 (d, J= 8.1 Hz, 1H, H-5), 8.27 (d, J= 8.1 Hz, 1H, H-7).

| | | | | |
|---|---|---|---|---|
| C₁₃H₁₂N₂O₄ (260.25) | Ber. | C 60.00 | H 4.65 | N 10.76 |
| | Gef. | 60.18 | 4.76 | 10.69 |

### Beispiel 3

### 4-Amino-1-(2,3-didesoxy-β-D-glycero-pentofuranosyl)-1H-indol

150 mg (0.58 mmol) der unter Bsp. 2c erhaltenen Verbindung werden in 15 ml EtOH gelöst und in Gegenwart von 15 mg Pd/C (10 % Pd) 12 h bei RT unter Atmosphärendruck hydriert. Man filtriert und dampft bis zur Trockene ein. Der Rückstand wird an Kieselgel, Rf (CH₂Cl₂/MeOH, 97:3)= 0.3) chromatographiert.
¹H NMR ([D₆]DMSO): δ= 1.91-2.40 (m, 4H, H-2' und H-3'), 3.49 (m, 2H, H-5'), 4.04 (m, 1H, H-4'), 4.81 (t, J= 5.5 Hz, 1H, 5'-OH), 5.20 (s, 2H, NH2), 6.15 (dd, J= 4.8 Hz und J= 6.6 Hz, 1H, H-1'), 6.20 (d, J= 7.5 Hz, 1H, H-5), 6.58 (d, J= 3.3 Hz, 1H, H-3), 6.71 (d, J= 7.5 Hz, 1H, H-7), 6.83 (t, 7.5 Hz, 1H, H-6), 7.32 (d, J= 3.3 Hz, 1H, H-2).

### Beispiel 4

### 1-(2,3-Didesoxy-β-D-glyceropentofuranosyl)-4-nitro-1H-indol

a) 5-O-[(1,1-Dimethylethyl)dimethylsilyl]-(2,3-didesoxy-α,β-D-glycero-pentofuranosyl)chlorid
5-O-[(1,1-Dimethylethyl)dimethylsilyl)-2,3-didesoxy-α,β-D-glycero-pentofuranose (1.51 g, 6.5 mmol) [M. Okabe, R.-C. Sun, S. Y.-K. Tam, L. T. Todaro, and D. L. Coffen , J. Org. Chem. 53, 4780, 1988] werden in 26 ml Tetrahydrofuran gelöst und mit CCl₄ (1 ml) unter N₂ versetzt. Man kühlt auf -80 °C und versetzt während 15 min tropfenweise mit Tris(dimethylamino)phosphin (1.56 ml). Nach ca. 2 h werden nochmals die gleichen Mengen CCl₄ und Phosphin hinzugefügt. Nach 6h zeigt das DC (Kieselgel, EtOAc-Petrolether, 2:8) einen etwa 50 proz. Umsatz des Lactols an. Die kalte Reaktionslösung der Halogenose wird direkt in die vorbereitete Glycosylierungsreaktion eingetragen.
b) 1-(2,3-Didesoxy-5-O-(tert-butyldimethylsilyl)-D-glyceropentofuranosyl)-4-nitro-1H-indol
1.0 g (6.16 mmol) 4-Nitroindol wird in 200 ml MeCN gelöst und zusammen mit 690 mg (12.32 mmol) KOH und TDA-1 20 Min. bei RT gerührt. Die in situ bereitete kalte Lösung der unter a) erhaltenen Halogenose (aus 12.32 mmol Lactol) wird der Suspension portionsweise zugespritzt und die Reaktionsmischung weitere 45 Min. intensiv gerührt. Unlösliche Bestandteile werden abfiltriert und das Filtrat zur Trockene eingedampft. Man adsorbiert an Kieselgel 60 (10 g) und chromatographiert an Kieselgel 60 H (Petrolether/EtOAc 8:2). Eindampfen der Hauptzone liefert ein Anomerengemisch in 60% Ausbeute, das aus 30% des β-Anomeren und 30% des α-Anomeren besteht. ¹H-NMR ([D₆]DMSO): δ (β-Anomer: 4.16 (m, 1H, H-4'), 6.42 (dd, J= 3.5 Hz und J= 6.4 Hz, 1H, H-1'); α-Anomer: 4.32 (m, 1H, H-4'), 6.47 (dd, H-1').

| | | | | |
|---|---|---|---|---|
| C₁₉H₂₈N₂O₄Si (376.53) | Ber. | C 60.61 | H 7.50 | N 7.44 |
| | Gef. | C 60.77 | H 7.42 | N 7.32 |

c) 1-(2,3-Didesoxy-βD-glycero-pentofuranosyl)-4-nitro-1H-indol
Das unter b) erhaltene β-Anomere wird in THF gelöst. Nach Zugabe von Bu4NF (1M Lösung in THF) rührt man 30 Min. bei Raumtemperatur. Das Lösungsmittel wird im Vakuum verdampft und der Rückstand an Kieselgel 60 adsorbiert. Nach Säulenchromatographie (CH₂Cl₂ - MeOH, 97:3) erhält man die Titelverbindung als gelbes Öl. R_{f} (CH₂Cl₂ - MeOH, 97:3)=0.4.
¹H-NMR ([D₆]DMSO): δ=4.11 (m, 1H, H-4'), 4.89 (t, J=5.4 Hz, 1H, 5'-OH), 6.41 (dd, J=4.0 und 6.7 Hz, 1H, H-1'), 7.09 (d, J=3.2 Hz, 1H, H-3), 7.37 (pt, J=8.1 Hz, 1H, H-6), 8.04 (d, J=3.2 Hz, 1H, H-2).

### Beispiel 4.1

### 1-(2,3-Didesoxy-α-D-glycero-pentofuranosyl)-4-nitro-1H-indol

Das unter 4b erhaltene α-Anomere wird analog zu Bsp. 4c desilyliert. Nach Säulenchromatographie (CH₂Cl₂ - MeOH, 97:3) erhält man die Titelverbinung als gelbes Öl, R_{f} =0.4.
¹H-NMR ([D₆]DMSO): δ = 4.27 (m, 1H, H-4'), 4,80 (t, J=5.7 Hz, 1H, 5'-OH), 6.49 (dd, J=4.2 Hz und 6.3 Hz, 1H, h-1'), 7.09 (d, J=3.2 Hz, 1H, H-3), 7.38 (pt, J=8.1 Hz, 1H, h-6), 7.93 (d, J=3.2 Hz, 1H,h-2).

## Patentansprüche

1. Arzneimittel enthaltend mindestens ein Nucleosid-Derivat der allgemeinen Formel I 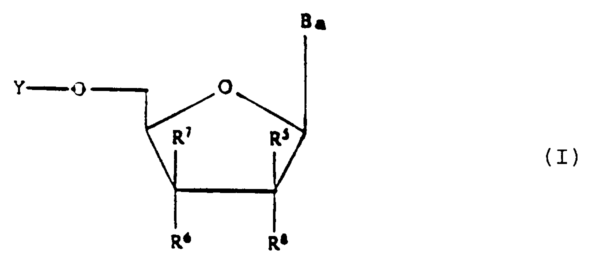 in der
Ba eine Indolylgruppe 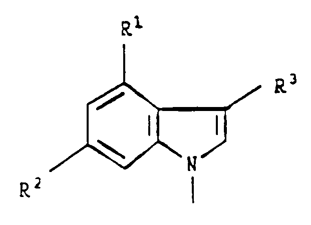 bedeutet, wobei
R¹ Wasserstoff, eine Amino-, C₁-C₆-Alkoxy-, Halogen- oder Nitrogruppe,
R² Wasserstoff oder eine Halogen- oder Aminogruppe,
R³ Wasserstoff
und
R⁵-R⁸ Wasserstoff oder R⁵ und R⁷ gemeinsam eine Bindung bilden
Y Wasserstoff oder eine C₁-C₇-Alkylcarbonyl-, Mono phosphat-,Diphosphat- oder Triphosphatgruppe darstellt
sowie deren Salze.

2. Arzneimittel gemäß Anspruch 1, dadurch gekennezeichnet, daß R¹ eine Amino- oder Nitrogruppe und R², R³, R⁵-R⁸ jeweils ein Wasserstoffatom, bzw. R⁵ und R⁷ zusammen auch eine Bindung darstellen.

3. Nucleosid-Derivate der allgemeinen Formel I 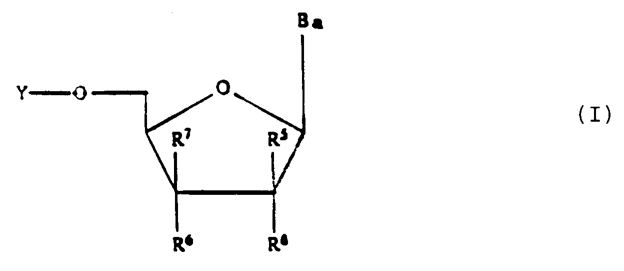 in der
Ba eine Indolylgruppe 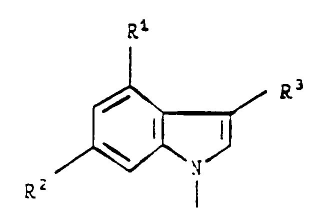 bedeutet, wobei
R¹ Wasserstoff, eine Amino-, C₂-C₆-Alkoxy-, Halogen oder Nitrogruppe
R² Wasserstoff, eine Halogen- oder Aminogruppe,
R³ Wasserstoff und
R⁵-R⁸ Wasserstoff oder R⁵ und R⁷ gemeinsam eine Bindung bilden und
Y Wasserstoff oder eine C₁-C₇-Alkylcarbonyl-, Mono-, Di- oder Triphosphatgruppe darstellt
sowie deren Salze.

4. Verfahren zur Herstellung der Verbindungen nach Anspruch 3, dadurch gekennzeichnet, daß man Verbindungen der Formel II
**Ba-X** (II)
in der
Ba eine Indolylgruppe 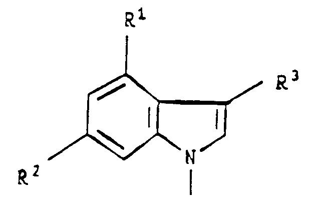 bedeutet, in der
R¹ Wasserstoff, eine Amino-, C₂-C₆-Alkoxy-, Halogen- oder Nitrogruppe,
R² Wasserstoff oder eine Halogen- oder Aminogruppe,
R³ Wasserstoff ist und
X ein Wasserstoffatom oder eine Alkalimetallgruppe darstellt,
mit einer Verbindung der Formel III 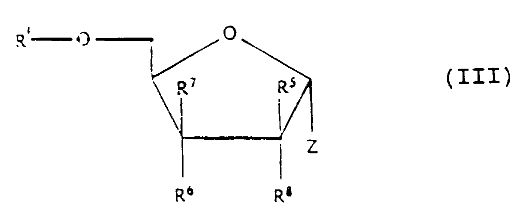 in der
R⁵-R⁸ Wasserstoff sind oder R⁵ und R⁷ gemeinsam eine Bindung bilden,
R' eine Sauerstoffschutzgruppe und
Z eine reaktive Gruppe bedeuten
zu Verbindungen der Formel IV 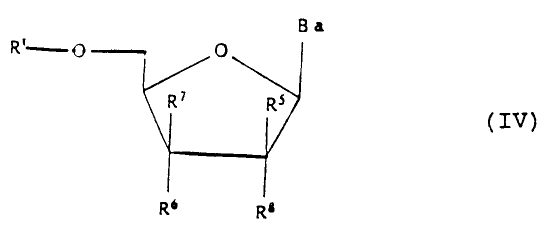 in der
Ba, R¹, R², R³, R⁵, R⁶, R⁷, R⁸ und R' die oben angegebene Bedeutung haben,
umsetzt und gegebenenfalls vorhandene Sauerstoffschutzgruppen abspaltet und gewünschtenfalls anschließend Verbindungen der Formel I, in denen Y Wasserstoff bedeutet, in bekannter Weise in die Mono-, Di- oder Triphosphate überführt und gewünschtenfalls erhaltene freie Basen bzw. Säuren in die entsprechenden Salze oder erhaltene Salze in die entsprechenden freien Basen bzw. Säuren umwandelt.

5. Verwendung von Verbindungen gemäß Anspruch 3 bei der DNA-Sequenzierung.

## Claims

1. Medicaments containing at least one nucleoside derivative of the general formula I 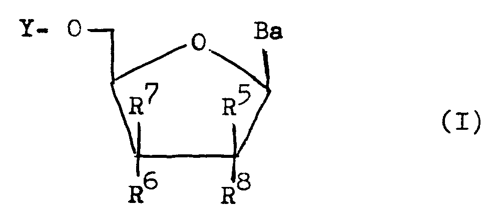 in which Ba signifies an indolyl group 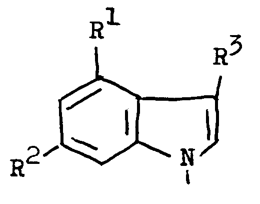 whereby R¹ represents hydrogen, an amino, C₁-C₆-alkoxy, halogen or nitro group, R² hydrogen or a halogen or amino group, R³ hydrogen and R⁵ - R⁸ hydrogen or R⁵ and R⁷ together a bond, Y hydrogen or a C₁-C₇-alkoxycarbonyl, monophosphate, diphosphate or triphosphate group, as well as their salts.

2. Medicament according to claim 1, characterised in that R¹ represents an amino or nitro group and R², R³, R⁵ - R⁸ each a hydrogen atom or R⁵ and R⁷ together also represent a bond.

3. Nucleoside derivatives of the general formula I 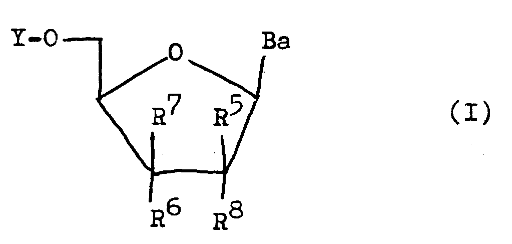 in which Ba represents an indolyl group 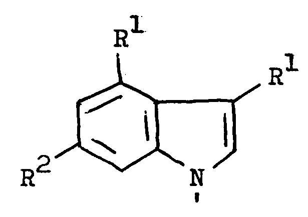 whereby R¹ represents hydrogen, an amino, C₁-C₆-alkoxy, halogen or nitro group, R² hydrogen, a halogen or amino group, R³ hydrogen and R⁵ - R⁸ hydrogen or R⁵ and R⁷ together form a bond and Y represents hydrogen or a C₁-C₇-alkylcarbonyl, mono-, di- or trophosphate group, as well as their salts.

4. Process for the preparation of the compounds according to claim 3, characterized in that one reacts compounds of the formula II
Ba-X (II)
in which Ba signifies an indolyl group. 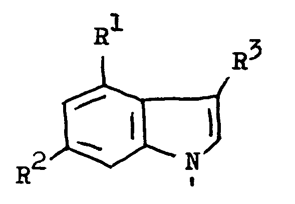 in which R¹ represents hydrogen, an amino, C₂-C₆-alkoxy, halogen or nitro group, R² hydrogen or a halogen or amino group, R³ hydrogen and X a hydrogen atom or an alkali metal group, with a compound of the formula III 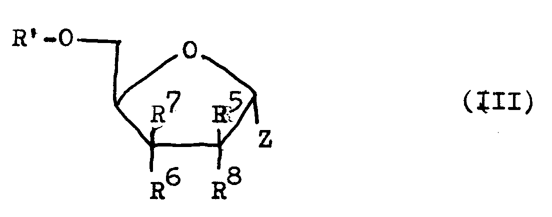 in which R⁵ - R⁸ are hydrogen or R⁵ and R⁷ together form a bond, R' signifies an oxygen protective group and Z a reactive group, to give compounds of the formula IV 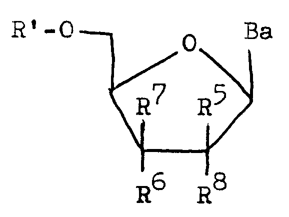 in which Ba, R¹, R², R³, R⁵, R⁶, R⁷, R⁸ and R' have the above-given meaning, and possibly splits off protective oxygen groups present and, if desired, subsequently converts compounds of the formula I, in which Y signifies hydrogen, in known manner into the mono-, di- or triphosphates and, if desired, converts free bases or acids obtained into the corresponding salts or converts salts obtained into the corresponding free bases or acids.

5. Use of compounds according to claim 5 in the case of DNA sequencing.

## Revendications

1. Médicament contenant au moins un dérivé de nucléoside de formule générale I 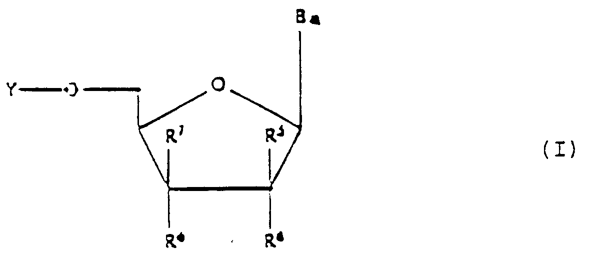 dans laquelle
Ba représente un groupe indolyle 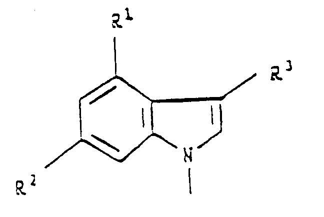 où
R¹ représente un atome d'hydrogène, un groupe amino, alcoxy en C₁-C₆, halogéno ou nitro,
R² représente un atome d'hydrogène ou un groupe halogéno ou amino,
R³ représente un atome d'hydrogène, et
R⁵-R⁸ représentent un atome d'hydrogène ou R⁵ et R⁷ forment ensemble une liaison,
Y représente un atome d'hydrogène ou un groupe alkylcarbonyle en C₁-C₇, monophosphate, diphosphate ou triphosphate,
ainsi que ses sels.

2. Médicament selon la revendication 1, caractérisé en ce que R¹ représente un groupe amino ou nitro et R², R³, R⁵-R⁸ représentent chacun un atome d'hydrogène, ou R⁵ et R⁷ forment ensemble également une liaison.

3. Dérivé de nucléoside de formule générale I 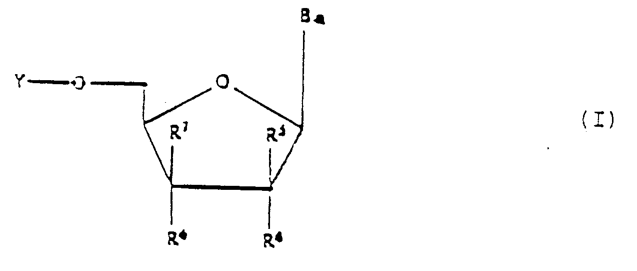 dans laquelle
Ba représente un groupe indolyle 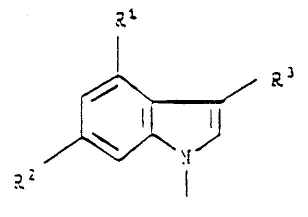 où
R¹ représente un atome d'hydrogène, un groupe amino, alcoxy en C₁-C₆, halogéno ou nitro,
R² représente un atome d'hydrogène ou un groupe halogéno ou amino,
R³ représente un atome d'hydrogène, et
R⁵-R⁸ représentent un atome d'hydrogène ou R⁵ et R⁷ forment ensemble une liaison,
Y représente un atome d'hydrogène ou un groupe alkylcarbonyle en C₁-C₇, monophosphate, diphosphate ou triphosphate,
ainsi que ses sels.

4. Procédé de préparation des composés selon la revendication 3, caractérisé en ce qu'on fait réagir des composés de formule II
Ba-X (II)
dans laquelle
Ba représente un groupe indolyle 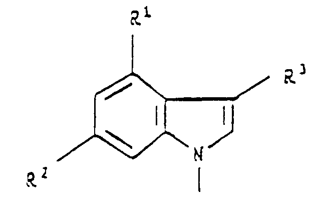 où
R¹ représente un atome d'hydrogène, un groupe amino, alcoxy en C₂-C₆, halogéno ou nitro,
R² représente un atome d'hydrogène ou un groupe halogéno ou amino,
R³ représente un atome d'hydrogène, et
X représente un atome d'hydrogène ou un groupe métal alcalin,
avec un composé de formule III 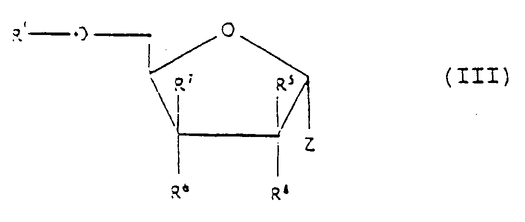 dans laquelle
R⁵-R⁸ représentent un atome d'hydrogène ou R⁵ et R⁷ forment ensemble une liaison,
R' représente un groupe protecteur d'oxygène et
Z représente un groupe réactif,
pour donner des composés de formule IV 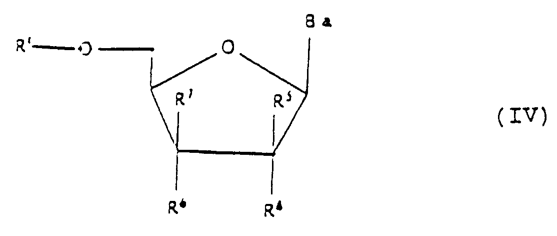 dans laquelle
Ba, R¹, R², R³, R⁵, R⁶, R⁷ R⁸ et R' ont les significations mentionnées ci-dessus,
et on sépare les groupes protecteurs d'oxygène éventuellement présents et ensuite, on transforme éventuellement de manière connue les composés de formule I, dans lesquels Y représente l'hydrogène, en mono-, di- ou triphosphates et éventuellement, on transforme les bases libres ou acides libres obtenus en les sels correspondants ou on transforme les sels obtenus en les bases ou acides libres correspondants.

5. Utilisation de composés selon la revendication 3, dans le séquençage d'ADN.
